# EUROPÄISCHE PATENTSCHRIFT

(11) **EP 1 424 115 B1**
(45) Veröffentlichungstag und Bekanntmachung des Hinweises auf die Patenterteilung: **13.01.2016**
(21) Anmeldenummer: 03405780.2
(22) Anmeldetag: 30.10.2003
(51) Int. Cl.: C07C 67/08, C07C 67/58, C07C 67/24, C07C 69/52

(54) **Verfahren zum Verestern einer Fettsäure**
Process for the esterification of a fatty acid
Procédé d'estérification d'un acide gras

(30) Priorität: 28.11.2002 EP 02406034
(43) Veröffentlichungstag der Anmeldung: 02.06.2004
(73) Patentinhaber: Sulzer Chemtech AG, 8404 Winterthur (CH)
(72) Erfinder: Moritz, Peter Dr., 8542 Wiesendangen (CH)
(74) Vertreter: Manitz, Finsterwald & Partner GbR

(56) Entgegenhaltungen:
- EP-A- 0 428 265
- EP-A- 0 474 996
- EP-A- 1 308 204
- WO-A-90/02603
- WO-A1-90/08121
- DE-A- 1 768 104
- DE-A- 19 829 809
- US-A- 6 069 261
- O. A. NEUMÜLLER: "Römpps Chemie-Lexikon" 1981, FRANCKH'SCHE VERLAGSHANDLUNG , STUTTGART , XP002239027 * Seite 1271 - Seite 1275 *
- KRAFCZYK J ET AL: "EINSATZ FON KATALYSATORPACKUNGEN FUER DIE HERSTELLUNG VON METHYLACETAT DURCH REAKTIVE REKTIFIKATION" CHEMIE. INGENIEUR. TECHNIK, VERLAG CHEMIE GMBH. WEINHEIM, DE, Bd. 66, Nr. 10, 1. Oktober 1994 (1994-10-01), Seiten 1372-1375, XP000467992 ISSN: 0009-286X

## Beschreibung

Die Erfindung betrifft ein Verfahren zum Verestern einer Fettsäure sowie eine Anlage zur Erzeugung von Fettsäureester durch eine heterogene Katalyse mit dem erfindungsgemässen Verfahren. Mit diesem Verfahren lässt sich eine der C12-, C14-, C16, C18-, C18'- oder C18"-Fettsäuren, nämlich die Laurinsäure, Myristinsäure, Palmitinsäure, Stearinsäure, Ölsäure oder Linolensäure mit einem der folgenden Alkoholen verestern: Methanol, Ethanol, n-Propanol, Isopropanol, n-Butanol, iso-Butanol bzw. 2-Ethyl-Hexanol.

Nach einem herkömmlichen Verfahren wird eine vorgegebene Menge an Fettsäure chargenweise verestert. Dabei wird die Fettsäure in einen beheizbaren Behälter mit Rührwerk gegeben und auf rund 100 bis 250°C aufgeheizt. Es wird ein Katalysator hinzu gegeben und unter einer kontinuierlichen Einspeisung von Alkohol die Veresterung bei konstant gehaltener Temperatur durchgeführt. Ein Dampfstrom aus dem Behälter wird in eine Trennkolonne eingeleitet, in der Wasser, das bei der Veresterung freigesetzt worden ist, von mitverdampften Alkohol getrennt wird. Das Wasser kann so aus dem Prozess entfernt werden, wodurch der Umsatz verbessert wird. Nach Abschluss der Veresterung werden durch weiteres Heizen die verbliebenen Reste an Alkohol und Wasser entfernt. In der US-A- 6 069 261 ist ein kontinuierliches Verfahren zum Verestern von Fettsäuren beschrieben, bei dem ein "permanentes" Hilfsgas verwendet wird um kontinuierlich Wasser zu entfernen. Ein solches Hilfsgas, das beispielsweise Wasserstoff oder Stickstoff ist, geht keine chemische Reaktion mit Reaktanden der Veresterung ein. In der WO 90/08121 ist ein kontinuierliches Verfahren zum Herstellen von Fettalkoholen beschrieben, bei dem eine Veresterung von Fettsäuren mit einem Alkohol in einer Reaktivkolonne stattfindet. Im unteren Teil dieser Kolonne wird gasförmiges Methanol zugegeben, welches als Strippgas im Gegenstrom nach oben steigt und das Reaktionswasser entfernt. Die Verwendung von gasförmigem Methanol hat verschiedene Nachteile, wie zum Beispiel einen erhöhten Energieverbrauch und ein erhöhtes Sicherheitsrisiko. Zudem ist die Wirkung des aufsteigenden gasförmigen Methanols auf den oberen Teil der Kolonne beschränkt und kann nicht auf den unteren Teil der Kolonne ausgeweitet werden.

Aufgabe der Erfindung ist es, ein weiteres Verfahren zum Verestern von Fettsäuren zu schaffen, das auch kontinuierlich durchführbar ist und bei dem die Nachteile zum Stand der Technik überwunden werden. Diese Aufgabe wird durch das im Anspruch 1 definierte Verfahren gelöst.

Das Verfahren zum Verestern einer Fettsäure F wird in einer Kolonne mit einer ein- oder mehrteiligen Packung durchgeführt. Die Packung hat zusätzlich zu einer Funktion als Reaktor die Funktion eines Abtriebteils. Sie ist zumindest in einem oberen Teil als eine Reaktionszone ausgebildet, in der eine heterogene Katalyse der Fettsäure F mit einem im gleichen molaren Einsatzverhältnis oder in einem Überschuss eingesetzten Alkohol A durchgeführt wird. Im Sumpf wird durch Verdampfen ein gasförmiger, alkoholreicher Gegenstrom erzeugt. Wasser wird aus der Reaktionszone mittels dem als Strippgas wirkenden Gegenstrom entfernt. Das beladene Strippgas wird am Kopf der Kolonne zumindest teilweise verflüssigt. Das Kopfprodukt wird in eine wasserreiche Fraktion sowie eine alkoholreiche Fraktion getrennt. Die alkoholreiche Fraktion wird als Ausgangsstoff für das Verestern und zur Erzeugung des Strippgases in das Verfahren rückgeführt. Für die Veresterung werden folgende Ausgangsstoffe als Fettsäure F bzw. Alkohol A verwendet: eine der C12-, C14-, C16-, C18-, C18'- oder C18"-Fettsäuren, nämlich Laurin-, Myristin-, Palmitin-, Stearin-, Öl- oder Linolensäure und einer der Alkohole Methanol, Ethanol, n-Propanol, Isopropanol, n-Butanol, Isobutanol oder 2-Ethyl-Hexanöl.

Die abhängigen Ansprüche 2 bis 8 betreffen vorteilhafte Ausführungsformen des Verfahrens gemäss Anspruch 1. Die Verwendung einer Anlage zum Durchführen dieses Verfahrens ist Gegenstand des Anspruchs 9.

Nachfolgend wird die Erfindung anhand der Zeichnungen erläutert. Es zeigen:
- Fig. 1: eine Anlage mit einer Kolonne, in der eine katalytische Reaktionszone angeordnet ist, die zur Durchführung des erfindungsgemässen Verfahrens verwendet wird,
- Fig. 2: einen Ausschnitt aus der katalytischen Reaktionszone,
- Fig. 3: einen Kopfbereich zu einer bevorzugten Ausführungsform der Kolonne und
- Fig. 4: eine Teilanlage zur Reinigung des erzeugten Fettsäureesters.

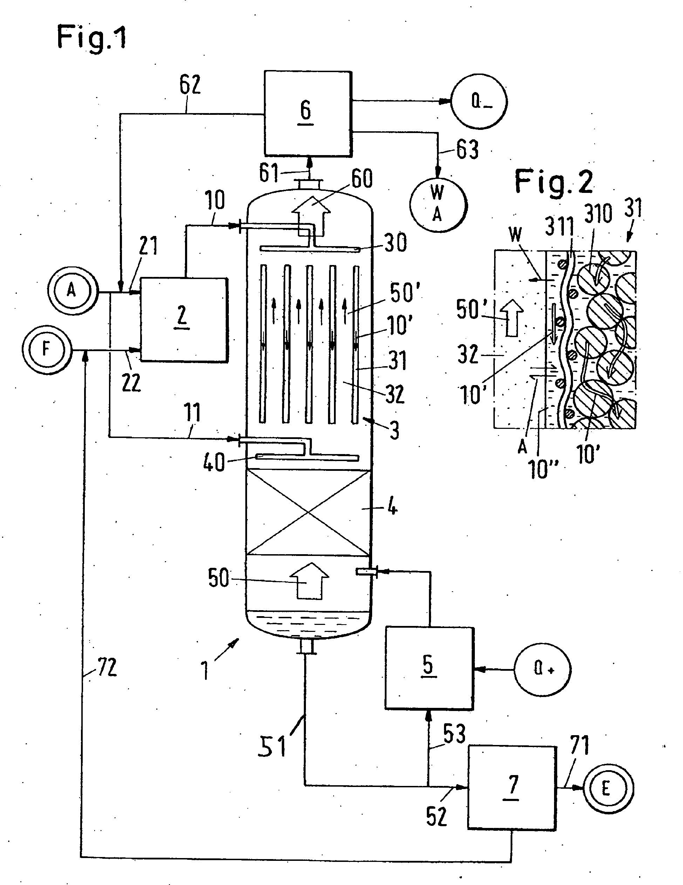

Die in Fig. 1 dargestellte Anlage umfasst eine Kolonne 1, aus deren Sumpf ein Zwischenprodukt, nämlich ein Gemisch, das den erfindungsgemäss erzeugten Fettsäureester enthält, über eine Leitung 51 entnommen und einer Teilanlage 7 zur Reinigung und Aufkonzentrierung zugeführt werden kann. Die vorliegende Beschreibung gilt für die Herstellung eines Fettsäureesters E, beispielsweise von Fettsäure-Isopropyl-Ester, der leichter als die korrespondierende Fettsäure F siedet.

Die Ausgangsprodukte des erfindungsgemässen Verfahrens sind der Alkohol A und eine Fettsäure F. Das in der Kolonne 1 gewonnene Zwischenprodukt enthält noch Reste der Ausgangsprodukte sowie Verunreinigungen. Das Zwischenprodukt wird in einer Trennkolonne der Teilanlage 7 weiter aufbereitet, so dass schliesslich ein Fettsäureester E gewonnen werden kann, dessen Reinheit mindestens 99 Gew-% beträgt. Das gereinigte Produkt wird durch die Leitung 71 abgezogen. Ein in der Trennkolonne abgetrennter Rest an Fettsäure F wird über eine Leitung 72 in das Verfahren rückgeführt.

Die Kolonne 1 enthält einen Packungsteil, der als eine katalytische Reaktionszone 3 ausgebildet ist, in welcher mittels einer heterogenen Katalyse eine Veresterung stattfindet. Durch eine Leitung 10 und einen Verteiler 30 wird ein Alkohol A und Fettsäure F enthaltendes Gemisch auf den Packungsteil 3 aufgebracht. Unter dem Packungsteil 3 ist ein zweiter Packungsteil 4 mit einem Verteiler 40 angeordnet. Über eine Leitung 11 und den Verteiler 40 wird zusätzlich Alkohol A in den Prozess eingespeist.

Die aus den beiden Teilen 3 und 4 bestehende Packung hat zusätzlich zur Funktion als Reaktor die Funktion eines Abtriebteils. Der Alkohol A wird im gleichen molaren Einsatzverhältnis oder in einem Überschuss eingesetzt. lm Sumpf kann daher durch Verdampfen ein gasförmiger, alkoholreicher Gegenstrom 50 erzeugt werden, der als Strippgas 50' wirkend Wasser W aus der Reaktionszone 3 entfernt. Die für das Verdampfen benötigte Wärme wird in einer Einrichtung 5 durch Heizen eines Zweigstroms 53 zugeführt (Wärmezufuhr Q₊). Bereits im unteren Teil 4 des Abtriebteils wird aus dem Flüssigkeitsgemisch, das aus der Reaktionszone 3 austritt, Wasser W und ausserdem auch Alkohol A von dem Strippgas 50 aufgenommen.

Das beladene Strippgas (Pfeil 60) wird am Kopf der Kolonne in einer Einrichtung 6 zumindest teilweise verflüssigt (Wärmeabfuhr Q -) und in zwei Fraktionen 62 und 63 getrennt. Die Fraktion 63 des Kopfprodukts ist wasserreich und hat einen Anteil an Wasser W von beispielsweise 80 Gew-%. Die alkoholreiche Fraktion 62 enthält beispielsweise 95 - 98 Gew-% an Alkohol A. Sie wird als Ausgangsstoff für das Verestern und zur Erzeugung des Strippgases 50 in das Verfahren rückgeführt. Die Trennung des Kopfprodukts in die alkoholreiche Fraktion 62 und die wasserreiche Fraktion 63 kann beispielsweise mittels eines Membranverfahrens, der Pervaporation, mittels eines Phasenscheiders oder mittels einer Destillationskolonne vorgenommen werden.

Der über den unteren Verteiler 40 eingespeiste Alkohol A wird auf der Packung 4 verdampft und trägt so zum Strom des Strippgases 50 bei. Die benötigte Verdampfungswärme gibt das Strippgas 50 ab, das beim Verdampfen im Sumpf in einen überhitzten Zustand gebracht wird.

Die katalytische Reaktionszone 3 setzt sich im gezeigten Beispiel aus vertikalen Lagen 31 und Strömungskanälen 32 für das Strippgas 50' zusammen. In und auf den Lagen 31 fliesst ein Gemisch 10', das die Ausgangsstoffe A und F enthält. Details zur Reaktionszone 3 sind im Ausschnitt der Fig. 2 dargestellt, nämlich eine Grenzregion zwischen einem Strömungskanal 32 mit dem Strippgas 50' und einer Lage 31, die ein Granulat 310 eines Katalysators enthält. Ein Gewebe 311 aus einem beständigen Material bildet Taschen, in denen das Granulat 310 eingeschlossen ist. Innerhalb einer Tasche und auf der äusseren Seite des Gewebes 311 fliesst das Gemisch 10', innerhalb der Tasche mit mäandrischen Strömungsfäden und ausserhalb als Rieselfilm. Durch die Oberfläche des Rieselfilms 10" tritt Wasser W in den Strom des Strippgases 50' über, da in diesem ein relativ kleiner Partialdruck des Wassers W vorliegt. Alkohol A wird in beiden Richtungen durch die Oberfläche des Rieselfilms 10" transportiert. Geeignete Packungen für die Reaktionszone 3 sind in der EP-A- 0 396 650 oder in der EP-A- 0 631 813 beschrieben.

Mit Vorteil werden die Ausgangsstoffe A und F in einem Vorreaktor 2 schon teilweise zur Reaktion gebracht, wobei 10 bis 30% der Fettsäure F in Ester E umgesetzt werden. Durch die Leitung 10 tritt somit ein Gemisch aus Alkohol A, Fettsäure F, Ester E und Wasser W in die Kolonne 1 ein. In der Reaktionszone 3 werden dann weitere 20 bis 70% der Fettsäure F umgesetzt.

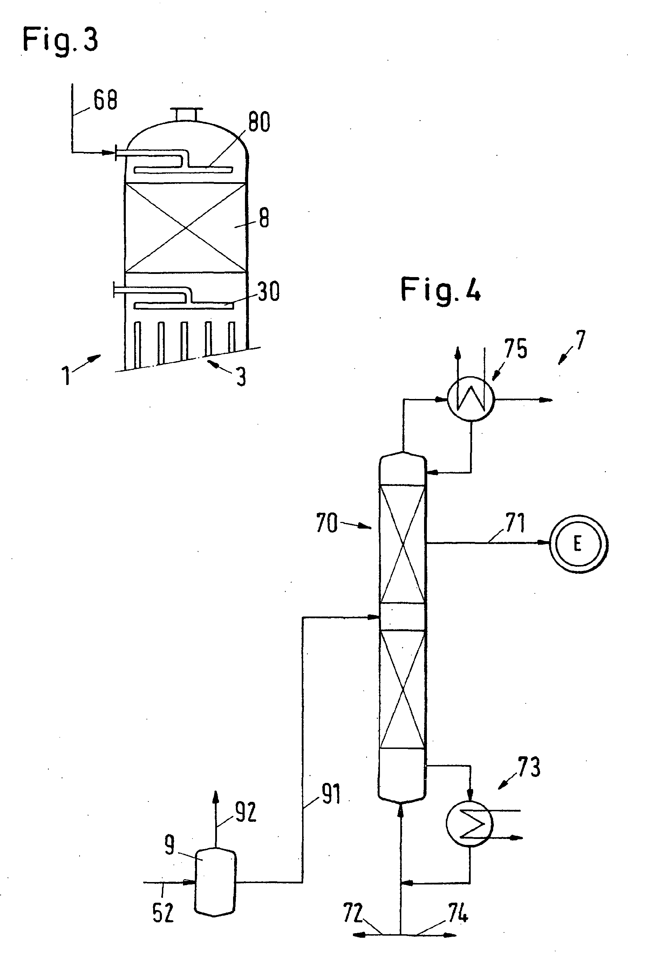

Mit Vorteil ist oberhalb des Abtriebteils 3, 4 eine weitere Packung 8 und ein Verteiler 80 - siehe Fig. 3 - in die Kolonne 1 eingebaut. In dieser Packung 8 wird mit einem Teil 68 der alkoholreichen und rückgeführten Fraktion 62 Fettsäure F, die das Strippgas 60 mitschleppt, abgefangen.

Fig. 4 zeigt die Teilanlage 7 zur Reinigung des erzeugten Fettsäureesters. Eine Komponente dieser Teilanlage 7 ist ein Entspannungsverdampfer 9, in der das Zwischenprodukt der Leitung 52 einen grossen Teil des restlichen Alkohols A in Dampfform freisetzt. Dieser alkoholreiche Dampf wird über eine Leitung 92 abgeführt. Er kann weiter für die Veresterung genutzt werden. Durch die Leitung 91 wird das alkoholarme Gemisch in eine Trennkolonne 70 geführt. Dort erfolgt die Entnahme des gereinigten Esters E (Leitung 71). Das aus einem Kondensator 75 durch eine Leitung 76 entnehmbare Kopfprodukt enthält Alkohol A und leichtflüchtige Verunreinigungen, insbesondere Luft. Aus dem Sumpf, dem mit einem Heizkreislauf 73 Wärme zugeführt wird, lassen sich Fettsäure F (Leitung 72) und schwerflüchtige Verunreinigungen (Leitung 74) abziehen. Die Teilanlage 7 kann aber auch als Destillationskolonne ausgeführt sein.

Anhand der Fig. 4 ist ein Trennverfahren beschrieben worden, das anwendbar ist, wenn der Fettsäureester E leichter als die korrespondierende Fettsäure F siedet. Bei Fettsäuren, die mit höheren Alkoholen (z.B. 2-Ethyl-Hexanol) verestert sind, liegen umgekehrte Verhältnisse bezüglich der Reinigung in der Teilanlage 7 vor. Der Fettsäureester wird im Sumpf der Trennkolonne 70 erhalten und muss weiter aufbereitet werden (nicht dargestellt). Fettsäure und verbliebener Alhohol, die sich im Kopf aus der Trennkolonne 70 entnehmen lassen, werden in das Verfahren rückgeführt.

Für die Veresterung werden folgende Ausgangsstoffe als Fettsäure F bzw. Alkohol A verwendet werden. Als F eine der C12-, C14-, C16-, C18-, C18'-oder C18"-Fettsäuren, nämlich Laurin-, Myristin-, Palmitin-, Stearin-, Öl- oder Linolensäure (engl. Lauric, Myristic, Palmitic, Stearic, Oleic oder Linoleic Acid); als A einen der Alkohole Methanol, Ethanol, n-Propanol, Isopropanol, n-Butanol, Isobutanol bzw. 2-Ethyl-Hexanol.

Als Katalysator lässt sich ein lonentauscherharz oder ein anorganischer Katalysator verwenden, wobei die Katalysatoren vorzugsweise in stark saurer Form vorliegen und das lonentauscherharz makroporös ausgebildet ist. Ist Methanol, Ethanol, n-Propanol, Isopropanol, n-Butanol oder Isobutanol der Alkohol A, so muss der Katalysator - in diesem Fall ein Ionenaustauscherharz - bei Temperaturen zwischen 120°C und 140°C verwendbar sein; in diesem Temperaturintervall darf er nur moderat an Aktivität einbüssen. Ist 2-Ethyl-Hexanol der Alkohol A, so muss der Katalysator - in diesem Fall ein anorganischer Katalysator - bei Temperaturen von 150-230°C eingesetzbar sein.

Nachfolgend werden für die Veresterung von Palmitinsäure (PA) mit Isopropanol (IPA) einige Werte von Verfahrensparametern angegeben.

Für die Ausgangsstoffe A = IPA und F = PA werden jeweils eine Reinheit von 95 bzw. 98% (Gew-%) bevorzugt. Es sind aber auch andere Werte möglich, nämlich in den Bereichen 88 - 100% für IPA und 95 - 100% für PA. Der Vorreaktor 2 wird bei 100°C und 2 bar betrieben. Er kann aber auch bei Temperaturen bis zu 140°C und entsprechend höheren Drücken bis zu 4 bar betrieben werden. Durch die Leitungen 21 und 11 werden in der Regel verschiedene Mengen an IPA eingespiesen. Das Verhältnis IPA(Leitung 21) zu IPA(Leitung 11) ist vorzugsweise 2.5, kann aber im Bereich von 1 bis 4 liegen. Der Kopfdruck in der Kolonne 1 beträgt 2 bis 5 bar und entsprechend die Temperatur in der Reaktionszone 3 120 bis 140°C. In der Heizeinrichtung 5 wird das Zwischenprodukt (Leitung 53) auf eine Temperatur zwischen 170 und 200°C erwärmt; es kann aber auch höher bis 250°C aufgeheizt werden.

Im Kopf der Kolonne oberhalb der Packung 8 (Fig. 3) liegt ein Strippgas 60 (Fig. 1) vor, das eine Temperatur zwischen 115 und 140°C hat und das 5 bis 12% Wasser W enthält. Das Zwischenprodukt in der Leitung 53 enthält 50 bis 80% Ester und 2 bis 10% IPA.

Eine Entspannungsverdampfung 9 wird bei 1 bar und 160 bis 210°C durchgeführt. Dabei verringert sich der Anteil an IPA auf rund 0.5 %. Die Trennkolonne 70 wird bei 15 bis 40 mbar betrieben. Im Kondensator 75 wird die Temperatur auf einen Wert zwischen 80 und 100°C reduziert und im Heizkreislauf 73 wird eine Temperatur zwischen 220 und 240°C erzeugt. Die obere Grenze dieser Temperatur darf auch bei 250°C liegen.

Nachfolgend werden zwei Beispiele zu erfindungsgemässen Veresterungen gegeben, die mit Pilotversuchen gewonnen worden sind.

Beispiel 1: Die Veresterung von Palmitinsäure mit Isopropanol wurde in einer Kolonne kontinuierlich bei atmosphärischem Druck unter folgenden Bedingungen durchgeführt:
Reaktionskolonne:
   Innerer Durchmesser: 50 mm.
   Verstärkungsteil und Abtriebsteil: Sulzer-Packung BX
   Reaktionszone: Katapak-S/ gefüllt mit stark saurem lonenaustauscher, der bis 140°C temperaturstabil ist
Feedströme:
   Palmitinsäure (Feed 1): 2.16 kg/h - Isopropanol (Feed 2): 1.44 kg/h
Produktströme:
   Kopfprodukt: 1.2 kg/h - Sumpfprodukt: 2.4 kg/h
Betriebsbedingungen:
   Kopfdruck: 1013 mbar - Rücklaufverhältnis: 0.2
   Temperatur in der Reaktionszone: ca. 98°C - Sumpftemperatur: 157.9 °C
Gemessene Sumpfkonzentrationen (in Gew.%): Isopropylpalmitat 14.06-Isopropanol: 10.61 - Palmitinsäure: 74.5-Wasser: 0.83

Beispiel 2: Der gleiche Aufbau wie in Beispiel 1 wurde in einer Druckkolonne realisiert. Dabei wurde ein bereits vorreagierter Feed eingesetzt, womit ein Vorreaktor simuliert werden konnte. Durch leichten Überdruck wurde in der Reaktionszone eine Temperatur im Bereich von der Reaktionszone von ca. 140°C eingestellt. Folgende Betriebsbedingungen führten zu einem deutlich höheren Reaktionsumsatz:
Feedströme:
   Feed 1: 2.3 kg/h mit folgender Zusammensetzung (in Gew.%):
      Isopropylpalmitat: 24.3 - Isopropanol: 21.6 - Palmitinsäure: 52.1 - Wasser: 2.0
      Feed 2: Isopropanol: 1.84 kg/h
Produktsröme:
   Kopfprodukt: 1.43 kg/h - Sumpfprodukt: 2.71 kg/h
Betriebsbedingungen:
   Kopfdruck: 5100 mbar- Rücklaufverhältnis: 0.2
   Temperatur in der Reaktionszone: ca. 140 °C - Sumpftemperatur: 192 °C
Gemessene Sumpfkonzentrationen (in Gew.%): Isopropylpalmitat 64.0 - Isopropanol: 7.7 - Palmitinsäure: 28.3 - Wasser: 0.06

## Patentansprüche

1. Verfahren zum Verestern einer Fettsäure F in einer Kolonne (1) mit einer ein- oder mehrteiligen Packung (3, 4), die zusätzlich zu einer Funktion als Reaktor die Funktion eines Abtriebteils hat und die zumindest in einem oberen Teil mit in der Packung fixierten Katalysatoren als Reaktionszone (3) ausgebildet ist,
in welchem Verfahren eine heterogene Katalyse der Fettsäure F mit einem im gleichen molaren Einsatzverhältnis oder in einem Überschuss eingesetzten Alkohol A durchgeführt wird,
im Sumpf durch Verdampfen ein gasförmiger, alkoholreicher Gegenstrom (50) erzeugt wird,
Wasser aus der Reaktionszone mittels dem als Strippgas wirkenden Gegenstrom entfernt wird,
das beladene Strippgas (60) am Kopf der Kolonne zumindest teilweise verflüssigt wird,
das Kopfprodukt in eine wasserreiche Fraktion (63) sowie eine alkoholreiche Fraktion (62) getrennt wird und
die alkoholreiche Fraktion als Ausgangsstoff für das Verestern und zur Erzeugung des Strippgases in das Verfahren rückgeführt wird **dadurch gekennzeichnet, dass** für die Veresterung folgende Ausgangsstoffe als Fettsäure F bzw. Alkohol A verwendet werden: eine der C12-, C14-, C16-, C18-, C18'- oder C18"-Fettsäuren, nämlich Laurin-, Myristin-, Palmitin-, Stearin-, Öl- oder Linolensäure und einer der Alkohole Methanol, Ethanol, n-Propanol, Isopropanol, n-Butanol, Isobutanol oder 2-Ethyl-Hexanol.

2. Verfahren nach Anspruch 1, **dadurch gekennzeichnet, dass** in einem unteren Teil (4) des Abtriebteils (3, 4) aus dem Flüssigkeitsgemisch, das aus der Reaktionszone (3) austritt, Wasser und Alkohol mit dem Strippgas (50) abgetrennt wird.

3. Verfahren nach Anspruch 1, **dadurch gekennzeichnet, dass** oberhalb des Abtriebteils (3, 4) eine weitere Packung (8) eingebaut ist, in der mit einem Teil (68) der alkoholreichen und rückgeführten Fraktion (62) Fettsäure, die das Strippgas (60) mitschleppt, abgefangen wird.

4. Verfahren nach einem der Ansprüche 1 bis 3, **dadurch gekennzeichnet, dass** in die Reaktionszone (3) oder unterhalb Alkohol eingespeist wird, der in verdampfter Form zum Strom des Strippgases (50, 50') beiträgt.

5. Verfahren nach einem der Ansprüche 1 bis 4, **dadurch gekennzeichnet, dass** die Trennung des Kopfprodukts in die wasserreiche Fraktion (62) und die alkoholreiche Fraktion (63) mittels Pervaporation, einem Phasenscheider oder einer Destillationskolonne vorgenommen wird.

6. Verfahren nach einem der Ansprüche 1 bis 5, **dadurch gekennzeichnet, dass** mit den Ausgangsstoffen A und F eine Teilveresterung in einem Vorreaktor (2) durchgeführt wird und das so gebildete Gemisch aus Fettsäure F, Alkohol A, Ester E und Wasser W in die Reaktionszone (3) der Kolonne (1) oder oberhalb aufgebracht wird.

7. Verfahren nach einem der Ansprüche 1 bis 6, **dadurch gekennzeichnet, dass** ein Teil (52) des flüssigen Sumpfgemisches (52) mittels einer Entspannungsverdampfung (9) oder Destillationskolonne und in einer Trennkolonne (70) weiter aufbereitet wird, so dass schliesslich ein Fettsäureester E gewonnen wird, dessen Reinheit mindestens 99 Gew-% beträgt.

8. Verfahren nach einem der Ansprüche 1 bis 7, **dadurch gekennzeichnet, dass** als Katalysator ein anorganischer Katalysator verwendet wird, der vorzugsweise bei Temperaturen von 150 bis 230°C einsetzbar ist, oder ein lonentauscherharz verwendet wird, das beim Durchführen des Verfahrens über 120°C und bis zu 140°C nur moderat an Aktivität einbüsst, wobei die Katalysatoren vorzugsweise in stark saurer Form vorliegen und das lonentauscherharz vorzugsweise makroporös ausgebildet ist.

9. Verwendung einer Anlage zur Erzeugung von Ester E durch eine heterogene Katalyse mit einem Verfahren gemäss einem der Ansprüche 1 bis 8, wobei die Anlage eine Kolonne (1) enthält mit einem Teil (3) einer Packung (3, 4) zum Durchführen der heterogenen Katalyse, welche Packung poröse Katalysatorträger (31) und Strömungskanäle (32) für das Strippgas (50') zwischen den Katalysatorträgern umfasst, wobei beim Durchführen des Verfahrens in Poren des Katalysatorträgers das zu behandelnde Gemisch einen abwärts gerichteten Flüssigkeitsstrom (10'), das Strippgas einen aufwärts gerichteten Strom bilden und die flüssige Phase mit der gasförmigen Phase in direktem Kontakt steht, so dass ein Stoffaustausch stattfinden kann.

## Claims

1. A method for the esterification of a fatty acid F in a column (1) having a single-part or multi-part packing (3, 4) which has the function of an output part in addition to the function as a reactor and which is configured as a reaction zone (3) in at least an upper part with catalysts fixed in the packing,
in which method a heterogeneous catalysis of the fatty acid F is carried out using an alcohol A used in the same molar use ratio or in a surplus;
a gaseous alcohol-rich counterflow (50) is produced by vaporisation in the sump;
water is removed from the reaction zone by means of the counterflow acting as a stripping gas;
the charged stripping gas (60) is at least partly liquefied at the head of the column;
the head product is separated into a water-rich fraction (63) and an alcohol-rich fraction; and
the alcohol-rich fraction is returned into the process as a starting material for the esterification and for the production of the stripping gas,
**characterised in that** the following starting materials are used as the fatty acid F or the alcohol A for the esterification: one of the fatty acids C12, C14, C16, C18, C18' or C18", namely lauric acid, myristic acid, palmitic acid, stearic acid, oleic acid or linoleic acid and one of the alcohols methanol, ethanol, n-propanol, isopropanol, n-butanol, isobutanol or 2-ethyl-hexanol.

2. A method in accordance with claim 1, **characterised in that** water and alcohol is separated out of the liquid mixture which leaves the reaction zone (3) using the stripping gas (50).

3. A method in accordance with claim 1 **characterised in that** a further packing (8) is installed above the outflow part (3, 4), in which packing fatty acid which the stripping gas (60) carries along is intercepted by a part (68) of the alcohol-rich and returned fraction (82).

4. A method in accordance with any one of the claims 1 to 3, **characterised in that** alcohol is fed into or beneath the reaction zone (3), said alcohol contributing to the flow of the stripping gas (50, 50') in vaporised form.

5. A method in accordance with any one of the claims 1 to 4, **characterised in that** the separation of the head product into the water-rich fraction (62) and the alcohol-rich fraction (63) is carried out by means of pervaporation, by means of a phase separator or by means of a distillation column.

6. A method in accordance with any one of the claims 1 to 5, **characterised in that** a part esterification is carried out in a pre-reactor (2) using the starting materials A and F and the mixture thus formed of fatty acid F, alcohol A, ester W and water W is applied into or above the reaction zone (3) of the column (1).

7. A method in accordance with any one of the claims 1 to 6, **characterised in that** a part (52) of the liquid sump mixture (52) is further treated by means of an expansion vaporisation (9) or by means of a distillation column and in a separation column (70) so that ultimately a fatty acid ester E is obtained whose purity amounts to at least 99% by weight.

8. A method in accordance with any one of the claims 1 to 7, **characterised in that** an inorganic catalyst is used as the catalyst which can preferably be used at temperatures from 150 to 230°C; or **in that** an ion exchange resin is used which only moderately loses activity on a carrying out of the method above 120°C and up to 140°C, wherein the catalysts are preferably present in a highly acidic form and the ion exchange resin is preferably macroporous.

9. Use of a plant for the production of ester E by a heterogeneous catalysis using a method in accordance with any one of the claims 1 to 8, wherein the plant includes a column (1) having a part (3) of a packing (3, 4) for carrying out the heterogeneous catalysis, which packing comprises porous catalyst carriers (31) and flow passages (32) for the stripping gas (50') between the catalyst carriers, wherein, on the carrying out of the method, the mixture to be treated forms a downwardly directed liquid flow (10') and the strip gas forms an upwardly directed flow in the pores of the catalyst carrier and the liquid phase is in direct contact with the gaseous phase such that a mass transfer can take place.

## Revendications

1. Procédé d'estérification d'un acide gras F dans une colonne (1) avec un garnissage (3, 4) en une ou plusieurs parties ayant une fonction de partie de stripage en plus d'une fonction de réacteur et réalisé au moins dans une partie supérieure en tant que zone de réaction (3) avec des catalyseurs fixés dans le garnissage,
une catalyse hétérogène de l'acide gras F étant effectuée dans le cadre dudit procédé avec un alcool A mis en oeuvre dans le même rapport molaire ou en excès,
un contre-courant (50) gazeux riche en alcool étant généré par évaporation dans le fond,
l'eau étant extraite de la zone de réaction au moyen du contre-courant agissant comme gaz de stripage,
le gaz de stripage (60) chargé étant au moins partiellement liquéfié en tête de colonne,
le produit de tête étant séparé en une fraction riche en eau (63) et en une fraction riche en alcool (62), et
la fraction riche en alcool étant recyclée comme substance initiale pour l'estérification et pour la génération du gaz de stripage du procédé, **caractérisé en ce que** les substances initiales suivantes sont utilisées comme acide gras F ou alcool A pour l'estérification : un des acides gras en C12, C14, C16, C18, C18' ou C18", en l'occurrence acide laurique, myristique, palmitique, stéarique, oléique ou linolénique, et un des alcools consistant en méthanol, éthanol, n-propanol, isopropanol, n-butanol, isobutanol ou 2-éthylhexanol.

2. Procédé selon la revendication 1, **caractérisé en ce que**, dans une partie inférieure (4) de la partie de stripage (3, 4), l'eau et l'alcool sont séparés avec le gaz de stripage (50) du mélange liquide sortant de la zone de réaction (3).

3. Procédé selon la revendication 1, **caractérisé en ce qu'**un autre garnissage (8) est intégré au-dessus de la partie de stripage (3, 4), dans lequel l'acide gras qui entraîne le gaz de stripage (60) est recueilli avec une partie (68) de la fraction (62) riche en alcool et recyclée.

4. Procédé selon l'une des revendications 1 à 3, **caractérisé en ce que** l'alcool est versé dans la zone de réaction (3) ou au-dessous de celle-ci, lequel contribue au flux du gaz de stripage (50, 50') sous sa forme évaporée.

5. Procédé selon l'une des revendications 1 à 4, **caractérisé en ce que** la séparation du produit de tête en fraction riche en eau (62) et en fraction riche en alcool (63) est effectuée par pervaporation, au moyen d'un séparateur de phase ou d'une colonne de distillation.

6. Procédé selon l'une des revendications 1 à 5, **caractérisé en ce qu'**une estérification partielle est effectuée dans un pré-réacteur (2) avec les substances initiales A et F et **en ce que** le mélange acide gras F, alcool A, ester E et eau W ainsi formé est refoulé dans la zone de réaction (3) de la colonne (1) ou au-dessus de celle-ci.

7. Procédé selon l'une des revendications 1 à 6, **caractérisé en ce qu'**une partie (52) du mélange liquide (52) continue à être traitée au moyen d'une vaporisation par détente (9) ou d'une colonne de distillation et dans une colonne de séparation (70), si bien qu'un ester d'acide gras E est finalement obtenu, dont la pureté est d'au moins 99 % en poids.

8. Procédé selon l'une des revendications 1 à 7, **caractérisé en ce qu'**un catalyseur inorganique est utilisé comme catalyseur, lequel est préférentiellement utilisable à température comprise entre 150 et 230°C, ou **en ce qu'**une résine échangeuse d'ions est utilisée, dont l'activité ne diminue que modérément lors de l'exécution du procédé au-dessus de 120°C et jusqu'à 140°C, les catalyseurs se présentant préférentiellement sous une forme fortement acide et la résine échangeuse d'ions étant préférentiellement macro-poreuse.

9. Utilisation d'une installation pour la production d'ester E par catalyse hétérogène avec un procédé selon l'une des revendications 1 à 8, ladite installation contenant une colonne (1) avec une partie (3) d'un garnissage (3, 4) pour l'exécution de la catalyse hétérogène, ledit garnissage comprenant des supports de catalyseur (31) poreux et des canaux d'écoulement (32) pour le gaz de stripage (50') entre les supports de catalyseur, où, lors de l'exécution du procédé, le mélange à traiter forme un courant de liquide (10') descendant dans les pores du support de catalyseur, le gaz de stripage forme un courant ascendant et où la phase liquide est en contact direct avec la phase gazeuse, de manière à permettre un échange de matière.
